# EUROPEAN PATENT SPECIFICATION

(11) **EP 0 460 076 B1**
(45) Date of publication and mention of the grant of the patent: **29.11.1995**
(21) Application number: 90904172.5
(22) Date of filing: 23.02.1990
(51) Int. Cl.: A61K 39/395, C07K 19/00, C12N 5/06, C12N 5/18

(54) **GENETICALLY ENGINEERED IMMUNOGLOBULINS**
GENTECHNOLOGISCH VERÄNDERTE IMMUNGLOBULINE
IMMUNOGLOBULINES AYANT SUBI UNE MANIPULATION GENETIQUE

(30) Priority: 24.02.1989 US 316144
(43) Date of publication of application: 11.12.1991
(73) Proprietor: THE REGENTS OF THE UNIVERSITY OF CALIFORNIA, Berkeley, California 94720 (US)
(72) Inventor: ZANETTI, Maurizio, La Jolla, CA 92037 (US); SOLLAZZO, Maurizio, La Jolla, CA 92037 (US)
(74) Representative: Kirschner, Klaus Dieter, Dipl.-Phys.
(86) International application number: PCT/US90/01010
(87) International publication number: WO 90/09804

(56) References cited:
- EP-A- 0 266 663
- GB-A- 2 188 638
- US-A- 4 631 191
- US-A- 4 694 072
- SCIENCE, vol. 229, 20 September 1985, US; MORRISON, pp. 1202-1207
- SCIENCE, vol. 227, 25 January 1985, US; WALDOR et al., pp. 415-417

## Description

### Field of the Invention

The present invention may utilize in its preferred embodiments, the use of recombinant DNA technology to genetically engineer natural or synthetically-derived immunoglobulin molecules, imparting therein novel epitopes, so as to create novel entities that can be employed in vitro and in vivo in a variety of means, such as to immunize against pathogens, and for example, build tolerance to antigens. In preferred embodiments, the epitopes are inserted into the so-called heavy or light chain variable domain of a given immunoglobulin molecule. Thus, known recombinant DNA technologies come to bear in the present invention, helping create novel immunoglobulin entities that retain functionality by localizing to particular cell types mechanistically via the so-called constant domains but otherwise functionally exploited to provide a novel localization of a particular antigenic determinant or epitope.

### Background of the Invention

Recombinant DNA technology has reached the point currently of being capable, in principle, of providing the methodology sufficient to identify, isolate and characterize DNA sequences, configure them for insertion into operative expression vectors and transfect those vectors variously into recombinant hosts such that those hosts are harnessed in their ability to produce the polypeptide encoded by the DNA sequence. Obviously, many variations attend the methodology associated with recombinant DNA technology, and particular means are not without inventive faculty. Nonetheless, methods are generally known in the published literature enabling requisite mental equipment for the art skilled to practice recombinant DNA technology in the production of polypeptides from a given recombinant host system.

Immunoglobulins (Igs) are the main effectors of humoral immunity, a property linked with their ability to bind antigens of various types. In view of the myriad numbers of antigens to a particular host organism, it can be appreciated that there are a like number or more of immunoglobulins that contain binding sites capable of specifically reacting with each antigen. These binding sites are located in the so-called variable region of the immunoglobulin and are referred to as the idiotype. In addition, immunoglobulin molecules are unique in their functionality of being capable of localizing to certain cell types, probably by means of mutual recognition of certain receptors that are located on the cell membrane. Immunoglobulins demonstrate a second general property whereby they act as endogenous modulators of the immune response. Igs and their idiotypic determinants have been used to immunize at the B- and/or T-cell level against a variety of exogenous antigens. In many cases, the immunity they evoke is comparable with that induced by the antigen itself. Although the principle underlying this phenomenon is understood, little is known about the molecular basis and the minimal structural requirements for the immunogenicity of Igs molecules and the interaction between those regions which may be responsible for such immunogenicity and the regions that are thought to provide the localization of a given immunoglobulin molecule with a particular cell/receptor type.

In the last many years, much progress has been made in endeavors to understand the immunogenic properties, structure and genetics of immunoglobulins. See Jeske, et al., Fundamental Immunology, Paul, ed., Raven Press, New York (1984), p 131 and Kabat, Journal Immunology 141, 525 (1988).

Initially, the antigenicity of the so-called variable (V) domain of antibodies was demonstrated. Oudin, et al., Academy of Sciences D 257, 805 (1963) and Kunkel, et al., Science 140, 1218 (1963). Subsequently, further research pointed out the existence of discrete areas of variability within V regions and introduced the notion of hypervariable (HV) or complementarity-determining regions (CDR). Wu, et al., J. Exp. Med. 132, 211 (1970). Many studies since have indicated that the immunogenic property of Ig molecules is determined presumably primarily by amino acid sequence contained in the CDRs. Davie, et al., Ann. Rev. Immunol. 4, 147 (1986). GB-A-2 188 638 and EP-A-0 266 663 disclose chimeric antibodies.

The basic immunoglobulin or antibody structural unit is well understood. The molecule consists of heavy and light chains held together covalently through disulfide bonds. The heavy chains are also covalently linked in a base portion via disulfide bonds and this portion is often referred to as the so-called constant region which is thought responsible for a given immunoglobulin molecule being mutually recognizable with certain sequences found at the surface of particular cells. There are five known major classes of constant regions which determine the class of the immunoglobulin molecule and are referred to as IgG, IgM, IgA, IgD and IgE. The N-terminal regions of the so-called heavy chains branch outwardly in a pictorial sense so as to give an overall Y-shaped structure. The light chains covalently bind to the Y branches of the two heavy chains. In the regions of the Y branches of the heavy chains lies a domain of approximately 100 amino acids in length which is variable, and therefore, specific for particular antigenic epitopes incidental to that particular immunoglobulin molecule.

It is to the Y branches containing the variable domains harboring the antigenic epitopes to which the particular attention is directed as a predicate of the present invention.

Prior researchers have studied and manipulated entire CDRs of immunoglobulins, producing chimeric molecules that have reported functionality. Exemplary attention is directed to Jones, et al., Nature 321, 522 (1986) reporting on a V-region mouse-human chimeric immunoglobulin molecule. This research thus amounted to a substantially entire CDR replacement as apparently does the research reported by Verhoeyen, et al., Science 239, 1534 (1988); Riechmann, et al., Nature 332, 323 (1988); and by Morrison, Science 229, 1202 (1985). See also European Patent Application Publication No. 125023A, published 14 November 1984.

Bolstered by the successful research summarized above that resulted presumably in functional chimeric molecules, the goal of the present research was to explore further the variable region contained in the N-terminus Y branches. It was a goal of the present research to manipulate these variable regions by introduction or substitution of novel determinants or epitopes so as to create novel immunoglobulin molecules that would possibly retain the localization functionality and yet contain functional heterologous epitopes. In this manner, the novel immunoglobulin molecules hereof could be employed for use within the organism at foreign sites, thereby imparting immunity characteristics in a novel site-directed manner. A problem facing the present researchers at that time lay in the fact that epitopes are found in a region of the Y branch. Therefore, it was difficult to envision whether any manipulation of the variable region would be possible without disrupting the interaction of heavy chain with the corresponding light chain, and if that proved inconsequential, whether the resultant molecule would retain its functionality, with respect to the novel epitope, in combination with the constant region of the basic immunoglobulin molecule. Thus, even hurdling the problem of where to experiment, it was not possible to predict whether one could successfully produce such novel, bifunctional immunoglobulin molecules.

The present research and invention are based upon the successful threshold experiment, producing model, novel immunoglobulin molecules found to be fully functional by virtue of their ability to localize on certain cell/receptor sites and retain specific reactivity of the introduced novel antigenic determinant or epitope.

### Summary of the Invention

The present invention is based upon the successful production of novel immunoglobulin molecules having introduced into the N-terminus variable region thereof a novel epitope not ordinarily found in the immunoglobulin molecule used as a starting molecule, such epitopes retaining specific reactivity. Preferably such reactivity is characterized by the epitope's ability to stimulate an antigenic response. Alternatively, such reactivity can reflect other specific biological functionality such as ligand or receptor binding.

The present invention is thus directed to novel immunoglobulin molecules having at least one novel heterologous epitope contained within the N-terminus variable domain thereof, said novel immunoglobulin molecule having retained functionality with respect to its C-terminus constant domain of the heavy chain specific for a particular cell/receptor type, and having novel, specific epitope in vitro and in vivo reactivity.

The present invention is further directed to pharmaceutical compositions containing, as essential pharmaceutical principle, a novel immunoglobulin hereof, particularly those in the form of an administrable pharmaceutical vaccine.

The present invention is further directed to methods useful for building tolerance to certain antigens, including those associated with autoimmune diseases, or for down-regulating hypersensitivity to allergens, or for providing active or passive immunity against certain pathogenic antigens, by administering to an individual in perceived need of such, a novel immunoglobulin molecule as defined above.

The present invention is further directed to novel recombinant means and methods useful for preparing, identifying and using the novel immunoglobulin molecules hereof including DNA isolates encoding them, vectors operatively harboring such DNA, hosts transfected with such vectors, cultures containing such growing hosts and the methods useful for preparing all of the above recombinant aspects.

### Detailed Description of the Invention

The present invention is described herein with particular detail for the preparation of model, novel immunoglobulin entities. This description is provided, as it was conducted, using recombinant DNA technology. Further detail herein defines methods by which one can test a given immunoglobulin to assure that it exhibits requisite functionality common to its starting material immunoglobulin and specially as to its novel epitopic antigenic activity. Given this information with respect to the particular novel immunoglobulin molecules described herein, coupled with general procedures and techniques known in the art, the art skilled will well enough know how to configure recombinant expression vectors for the preparation of other novel immunoglobulin molecules falling within the general scope hereof for use as herein described. Thus, having described the threshold experiment of the successful preparation of a novel immunoglobulin molecule, one skilled in the art need not follow the exact details used for reproducing the invention. Instead, the art skilled may borrow from the extant, relevant art, known techniques for the preparation of still other novel immunoglobulin molecules falling within the general scope hereof.

### 1. Figure Legends

Figure 1 is a diagram illustrating the construction of the pNy1NANP expression vector.

Figure 2 is an SDS-PAGE of the y1NANP and WT recombinant Ig.

Figure 3 shows the binding of ¹²⁵I-labelled monoclonas antibody Sp-3-B4 to engineered antibody y1NANP.

Figure 4 is a western blot binding of ¹²⁵I-labelled antibody Sp3-B4 to engineered antibody y1NANP and localization of the engineered (NANP)₃ epitope in the H chain.

Figure 5 shows results of cross-inhibition of ¹²⁵I-labelled antibody Sp3-B4 binding to synthetic peptide (NANP)₃ (panel A) or engineered antibody y1NANP (panel B) by y1NANP Ig or peptide (NANP)₃.

### 2. General Methods and Definitions

"Expression vector" includes vectors which are capable of expressing DNA sequences contained therein, where such sequences are operatively linked to other sequences capable of effecting their expression. It is implied, although not always explicitly stated, that these expression vectors may be replicable in the host organisms either as episomes or as an integral part of the chromosomal DNA. "Operative," or grammatical equivalents, means that the respective DNA sequences are operational, that is, work for their intended purposes. In sum, "expression vector" is given a functional definition, and any DNA sequence which is capable of effecting expression of a specified DNA sequence disposed therein is included in this term as it is applied to the specified sequence. In general, expression vectors of utility in recombinant DNA techniques are often in the form of "plasmids" referred to as circular double stranded DNA loops which, in their vector form, are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

Apart from the novelty of the present invention involving the introduction of novel epitopes by means of repositioning or augmentation of a parent immunoglobulin, it will be understood that the novel immunoglobulins of the present invention may otherwise permissively differ from the parent in respect of a difference in one or more amino acids from the parent entity, insofar as such differences do not lead to a destruction in kind of the basic activity or bio-functionality of the novel entity.

"Recombinant host cells" refers to cells which have been transfected with vectors defined above.

Extrinsic support medium is used to support the host cells and includes those known or devised media that can support the cells in a growth phase or maintain them in a viable state such that they can perform their recombinantly harnessed function. See, for example, ATCC Media Handbook, Ed. Cote et al., American Type Culture Collection, Rockville, MD (1984). A growth supporting medium for mammalian cells, for example, preferably contains a serum supplement such as fetal calf serum or other supplementing component commonly used to facilitate cell growth and division such as hydrolysates of animal meat or milk, tissue or organ extracts, macerated clots or their extracts, and so forth. Other suitable medium components include, for example, transferrin, insulin and various metals.

The vectors and methods disclosed herein are suitable for use in host cells over a wide range of prokaryotic and eukaryotic organisms.

As used herein "epitope" is a moiety capable of eliciting specific epitopic reactivity, preferably specific antigenic reactivity or domain binding functionality.

"Heterologous" with reference herein to the novel epitope for a given immunoglobulin molecule refers to the presence of (at least one) such epitope in the N-terminus domain of an immunoglobulin that does not ordinarily bear that epitope(s) in its native state and/or do not themselves constitute all or part of the CDR of an immunoglobulin. Hence, that chain contains heterologous epitope sequence(s). Such heterologous epitope sequences shall include the classic antigenic epitopes as well as receptor like binding domains or binding regions that function as receptor sites, such as the human CD4 binding domain for HIV, hormonal receptor binding ligands, retinoid receptor and ligands or receptors that mediate cell adhesion.

"Chimeric" refers to immunoglobulins hereof, bearing the heterologous epitope(s), that otherwise may be composed of parts taken from immunoglobulins of more than one species. Hence, a chimeric starting immunoglobulin hereof may have a hybrid heavy chain made up of parts taken from corresponding human and non-human immunoglobulins.

In addition to the above discussion and the various references to existing literature teachings, reference is made to standard textbooks of molecular biology that contain definitions and methods and means for carrying out basic techniques encompassed by the present invention. See, for example, Maniatis, et al, Molecular Cloning: A Laboratory Manual, Cold Spring Harbor Laboratory, New York, 1982 and the various references cited therein, and in particular, Colowick et al., Methods in Enzymology Vol 152, Academic Press, Inc. (1987).

The foregoing description and following experimental details set forth the methodology employed initially by the present researchers in identifying and characterizing and preparing particular immunoglobulins. The art skilled will recognize that by supplying the present information including the wherewithal of the location and makeup of the epitope containing domain of a given immunoglobulin, and how it can be manipulated to produce the novel immunoglobulins hereof. Therefore, it may not be necessary to repeat these details in all respects in their endeavors to reproduce this work. Instead, they may choose to employ alternative, reliable and known methods, for example, they may synthesize the underlying DNA sequences encoding a particular novel immunoglobulin hereof for deployment within similar or other suitable, operative expression vectors and culture systems. Thus, in addition to supplying details actually employed, the present disclosure serves to enable reproduction of the specific immunoglobulins disclosed and others, and fragments thereof, such as the individual chains for in vitro assembly, using means within the skill of the art having benefit of the present disclosure. All of such means are included within the enablement and scope of the present invention.

### 3. Description of Particularly Preferred Embodiments

Protein engineering was used to introduce a foreign epitope into the CDR3 of the H chain of a mouse/human chimeric antibody (C_{γ1}62). This epitope consists of three copies of the tetrapeptide Asn-Ala-Asn-Pro (NANP). The tetrapeptide occurs naturally as a 37 tandem repeat in the Plasmodium falciparum circumsporozoite (CS) protein, interspersed with four repeats of the variant sequence Asn-Val-Asp-Pro [Dame et al., Science 225, 593 (1984)]. In the construct described here, the epitope is flanked by Val and Pro residues at each end [VP (NANP)₃ VP]. The experiment verified that the (NANP)₃ epitope could be inserted in the HV region of a host H chain (V_{H}) without altering the framework folding of the Ig molecule, i.e., its molecular assembly with the light (L) chain and it determined that the antigenic and immunogenic properties of the recombinant Ig molecule were expressed. It is known that the CDR3 of V_{H} regions of antibody is often the structural correlate of an immunodominant idiotope [Davie, et al, Ann. Rev. Immunol. 4, 147 (1986)], which indicated that the CDR3 is at the surface of the molecule. Moreover, it is well established that because of recombination of the variable-diversity-joining (VDJ) regions, as well as N-addition mechanisms [Tonegawa, Nature 302, 575 (1983); Miller et al., Immunol. Today 7, 36 (1986)], the CDR3 may vary considerably in length (from 3 to 19 amino acids) [Kabat, et al.., Proteins of Immunological Interest, U.S. Dept. of Health and Human Service NIH (1987)], implying a high degree of plasticity at the structural level. Second, the (NANP)₃ epitope selected for this study is relatively short, repetitive and of proven immunogenicity in mice and humans [Good et al., Ann. Rev. Immunol. 6, 663 (1988)].

### 4. Examples

### EXAMPLE I

The production of hybridoma 62 and B10H2, and the purification of mAb 62 and 109.3 (anti-2,4-dinitrophenol) have been described previously [Zanetti et al., J. Immunol. 131, 2452 (1983) and Glotz et al., J. Immunol. 137, 223 (1986)].

A DNA library was constructed from size-selected 2-2.5-kb Eco RI fragments from hybridoma 62 genomic DNA. Fragments were eluted from low melting point agarose and ligated into the λgt10 vector [Huynh et al., DNA Cloning Techniques 1, 49 (1985)]. After ligation and packaging, 5 x 10⁴ plaque-forming units were screened by replicate hybridization with the J_{H} [Sakano et al., Nature 286, 676 (1980)] and pSAPC15 [Brodeur et al., Eur. J. Immunol. 14, 922 (1984)] probes. Four clones were isolated and plaque purified; the 2.3-kb EcoRI insert form one of them was subcloned into pEMBL18 vector [Dente et al., DNA Cloning Techniques 1, 101 (1985)]. The V_{H}B10H2 coding sequence was determined by cloning the cDNA from the parental hybridoma by primer extension of the poly(A)⁺ RNA with a synthetic oligonucleotide (5'GGGGCCAGTGGATAGAC3') that anneals at the 5' end of the CH1 region. The same oligonucleotide was used as a probe for screening the library after 5' end-labeling by kinase with ³²P-ATP. The nucleotide sequence of both clones was determined by dideoxy method on both strands after subcloning suitable restriction fragments into the pEMBL18 vector.

Plasmid pN_{γ1}62 containing DNA encoding C1,62 antibody was constructed by subcloning in the proper orientation the 2.3-kb EcoRI DNA fragment carrying the V_{H}62 rearrangement into the unique EcoRI site of the PN_{γ1} Sollazo et al., Focus 10, 64(1988) vector (a PSV derived vector harboring an human γ1, gene). This vector encodes a human γ₁ gene downstream from the EcoRI site. It also carries a neomycin resistance gene under the control of the SV40 promoter for the selection of stable transformant cells. Transfectoma cells were constructed by introducing the plasmids pN_{γ1}62 and pN_{γ1}CHA, a chimeric construct encoding an antibody lacking Id62* and Ig binding into J558L mouse by electroporation. This cell line is an H chain-defective variant of myeloma J558 [Morrison et al., Science 229, 229 (1985)] and carries the rearrangement for a λ1 light (L) chain. Briefly 3 x 10⁶ cells in 1 ml of Dulbecco's modified minimum essential medium (DMEM) containing 10 »g of supercoiled plasmid DNA were pulsed for 17 ms at 650 V/cm in a Cell Porator apparatus (Bethesda Research Laboratories, Bethesda, MD). After pulsing, the cells were resuspended in 10 ml of DMEM supplemented with 10 mM Hepes buffer, 2 mM L-glutamine, penicillin (50 »g/ml), streptomycin (50 »g/ml) and 10% fetal calf serum (cDMEM), and incubated for 48 h at 37°C in a 10% CO₂ atmosphere. The cells were then resuspended in 20 ml of cDMEM and an aliquot (2 ml) was diluted into 20 ml of cDMEM containing 1.2 mg/ml of G418 (Gibco, Grand Island, NY), plated on a 96-well microtiter plate and cultured for 14 days. The supernatants of neomycin-resistant colonies (stable transformants) were tested by solid-phase radioimmunoassay (RIA) and enzyme-linked immunosorbent assay (ELISA).
* The term "Id62" refers to "idiotype number 62" which is a laboratory designation for the idiotype of the antibody produced by the hybridoma "62" described in Example I. The term "Ig binding" denotes immunoglobulin binding.

The presence of Id62 in the supernatant of J558L cells transfected with pN_{γ1}62 vector was tested by competitive inhibition in ELISA. This measures the inhibition (percent) of the binding of horseradish peroxidase (HP)-conjugated mAb 62 (ligand) to anti-Id62 antibody coated on 96-well polyvinyl microtiter plate (Dynatech, Alexandria, VA) [Zanetti et al., J. Immunol. 131, 2452 (1983)]. The supernatant of J558L cells transfected with pN_{γ1}CHA plasmid and purified mAb 62 and 109.3 (an IgG₁, x anti-2,4-dinitrophenol) served as controls [Zanetti et al., J. Immunol. 131, 2452 (1983)]. A second method to test for Id62 expression was by Western blot [Towbin et al., Proc. Natl. Acad. Sci. USA 71, 4350 (1979)]. Briefly, approximately 5 »g of antibody C_{γ1}62 purified by affinity chromatography on an anti human Ig Sepharose 4B column (Pharmacia, Uppsala, Sweden) was electrophoresed on a 10% sodium dodecyl sulfate polyacrylamide gel electrophoresed on a 10% sodium dodecyl sulfate polyacrylamide gel electrophoresis under reducing conditions. The gel was then blotted onto 0.45 M nitrocellulose paper (Millipore, Bedford, MA) and probed with ¹²⁵I-labelled affinity-purified syngeneic anti-Id62 antibody [Zanetti et al. J. Immunol. 135, 1245 (1985)]. Antibodies 62 and 109.3 served as positive and negative control, respectively. The filter was exposed a first time for 24 h at -70°C with intensifier screen. To demonstrate the co-expression of the human C region on the H chain of the chimeric C_{γ1}62 antibody, the nitrocellulose paper was re-probed with ¹²⁵I-labelled goat anti-human Ig antibody and exposed for 2 h at 70°C.

Sequence data is publicly available from EMBL/Gene Bank Data Library under Accession No. Y00744.

The _{γ1}NANP antibody carrying the malarial CS immunodominant B-cell epitope NANP in the CDR3 of its H chain was engineered (antigenized) as follows:

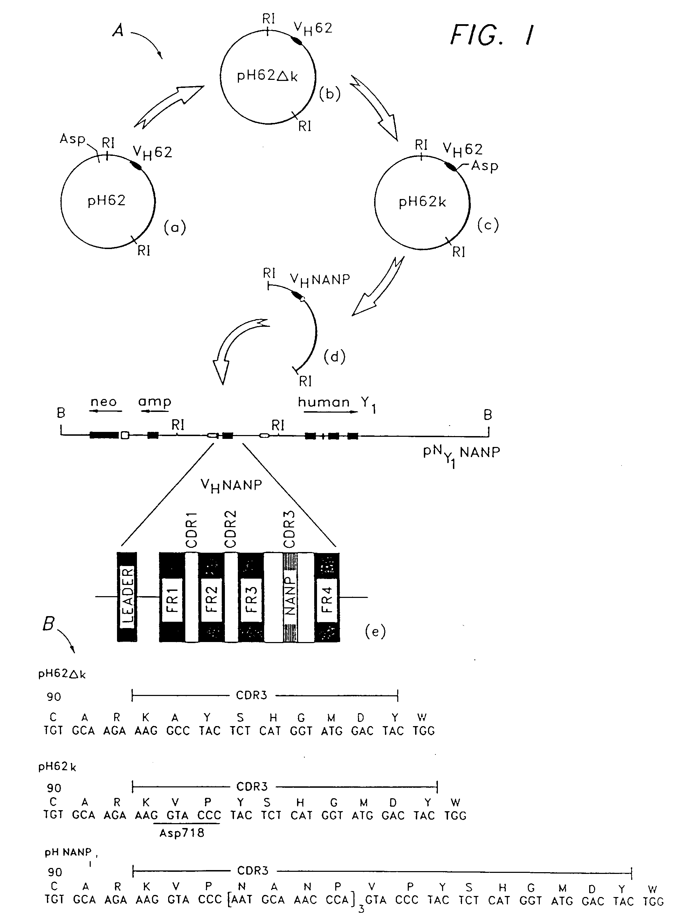

Figure 1 is a diagram illustrating the construction of the pNy1NANP expression vector. In panel A: (a) The productively rearranged V_{H} gene of the hybridoma cell line 62 isolated from a size-selected lambda gt10 library and subcloned into pBluescript (publicly available from Stratagene, San Diego, CA) is described infra.; (b) The restriction site Kpn I/Asp718 of the polylinker region was deleted by Kpn I digestion, filled in with T4 polymerase and ligated, yielding the plasmid pH62Δk; (c) pH62Δk was used as a template for site-directed mutagenesis to introduce a unique Asp718 restriction site in CDR3 of the V_{H} gene. The synthetic oligonucleotide (5'CAAGAAAGGTACCCTACTCTC 3'), which encodes a 3 bp insertion (TAC), was annealed to the uracylated single-stranded complementary template and elongated; (d) Complementary synthetic oligonucleotides 
(5'GTACCCAATGCAAACCCAAATGCAAACCCAAATGCAAACCCA 3'
    3'GGTTACGTTTGGGTTTACGTTTGGGTTTACGTTTGGGTCATG 5')
were annealed and subcloned into the unique Asp718 site of pH62k. The construction was verified by sequence analysis by using a 15^{mer} primer corresponding to the 5' end of V_{H}62 gene (5'GACGTGAAGCTGGTG 3'); (e) The 2.3-kb Eco RI fragment carrying the engineered V_{H}NANP gene was subcloned upstream from the human y1 C region into the 15-kb pNy1 vector. The pNy1NANP construct was electroporated into J558L cells subsequently cultured in the presence of G418. Resistant clones were screened for Ig production by a sandwich enzyme-linked immunosorbent assay (ELISA) using goat anti-human antibodies immobilized on microtiter wells as the capturing antibodies and horseradish peroxidase (HP) conjugated goat anti-human Ig (Sigma) as the revealing antibodies. Clones producing >2-5 »g Ig/ml of protein 10⁶ cells were expanded and the antibody purified from culture supermatants. Sequence modifications illustrated in panel A are shown in detail in panel B. Abbreviations used: Asp - Asp 718; B - Bam HI; RI - Eco RI; FR - framework region; CDR - complementarity-determining region; neo - neomycin (G418) resistance; amp - ampicillin resistance.

The restriction fragment encoding the V_{H} gene of a murine monoclonal antibody to thyroglobulin (mAb 62) was modified as shown in Figure 1. A double-stranded synthetic DNA fragment encoding three copies of the NANP tetramer (NANP)₃ and carrying Asp718 protruding ends was inserted in frame between Pro 95 and Tyr 96 of V_{H}62k coding region. The pH62NANP construct was verified by dideoxy sequencing. The Eco RI restriction fragment encoding the engineered V_{H} was subcloned into the pNY₁ expression vector upstream from the human Y₁ constant (C) region to obtain the pNY₁NANP construct. This plasmid was electroporated into the murine J558L cell line, a H chain-defective variant of myeloma J558 that carries the rearrangement for a lambda-1 L chain [Morrison et al., Science 229, 1202 (1985)].

Transfectoma cells were cultured, subcloned and screened for secretion of the engineered (antigenized) Ig molecule using a sandwich enzyme-linked immunosorbent assay (ELISA) with goat anti-human Ig antibodies. Clones producing 2-5 »g/ml of protein 10⁶ cells were selected and expanded, and the chimeric protein was purified by means of affinity chromatography on a Sepharose 4B-protein-A column. The purified Ig molecule was analyzed by SDS-PAGE under reducing and nonreducing conditions.

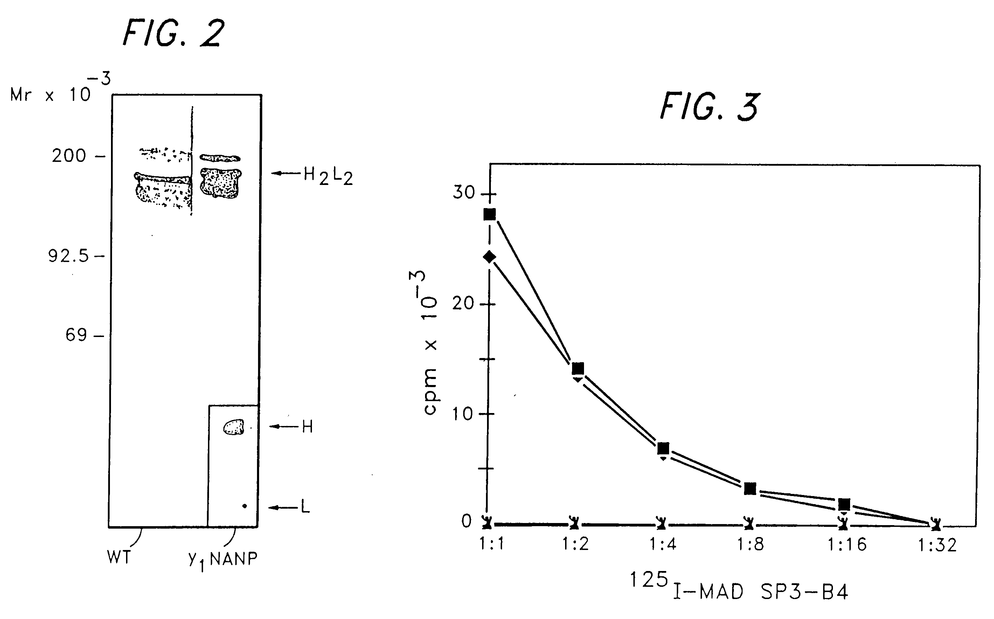

Figure 2 is an SDS-PAGE of the y1NANP and WT recombinant Ig. Five g of Protein A-purified antibody were loaded on a 7.5% polyacrylamide gel under nonreducing conditions. The gel was stained with Comassie blue. The inset shows the resolution into heavy (H) and light (L) chains of engineered (antigenized) antibody y1NANP electrophoresed on a 10% polyacrylamide gel under reducing (5% β-mercaptoethanol) conditions.

Figure 2 shows that the nonreduced y1NANp chimeric antibody has an apparent molecular weight of 160 kD, suggesting a proper H₂L₂ assembly to form a tetrameric Ig protein. When the y1NANP antibody was compared with the wild-type (WT) Ig, a chimeric antibody lacking the (NANP)₃ insert, purified from culture supernatant fluid of J558L cells transfected with pNY₁62, a slight difference in size was observed due to the presence of the inserted epitope. However, the molecular weight of the y1NANP antibody is well in the range of a tetrameric complex. Both preparations also showed a smear in the region below the 160 kD band, suggesting some degradation and/or noncorrectly assembled protein products. Under reducing conditions, the engineered (antigenized) Y₁NANP antibody was appropriately resolved into an H and na L chain (Figure 2, inset). As determined by ELISA of NP-40 lysates, transfectoma cells secreting the Y₁NANP antibody had approximately the same cytoplasmic levels of H chains as cells producing the WT Ig. Collectively, these results indicate that the insertion of 15 amino acids into the CDR3 of V_{H}62 did not appreciably alter the interaction between V_{H} and V_{L} polypeptide chains nor the assembly and secretion of the tetrameric (H₂L₂) Ig molecule.

To determine if the engineered (antigenized) Y₁NANP antibody indeed expresses the (NANP)₃ epitope in an immunological accessible form, solid-phase radioimmunoassay (RIA) and Western blot techniques were used and a murine monoclonal antibody (Sp3-B4) generated against P. falciparum and specific the NANP epitope.

Figure 3 shows the binding of ¹²⁵I-labelled monoclonal antibody Sp-3-B4 to engineered (antigenized) antibody Y₁NANP. Murine monoclonal antibody (mAb) Sp3-B4, an IgG2a,k antibody produced by immunization with the P. falciparum parasite and reacting with the repetitive epitope NANP. Specific for the NANP epitope, any antimalainal antibody could be so used as a tool and generated via analogous techniques. Polyvinyl microtiter wells were coated by drying at 37°C with 5 g/ml solution in 0.9% NaCl of purified y1NANP Ig (solid diamonds), WT (solid triangles) (NANP)₃ synthetic peptide (solid squares), a 16^{mer} synthetic peptide (YYCARKAYSHGMDYW) encompassing the CDR3 of the V_{H} region of prototype antibody 62 (open squares), and the 15^{mer} synthetic peptide YPQVTRGDVFTMPED of the cell-adhesive molecule vitronectin (open diamonds). The ¹²⁵I-labelled antibody Sp3-B4 (20 x 10⁴ cpm/50»l) was incubated overnight at +4°C. After extensive washing, the bound radioactivity was counted in a gamma counter. The test was done in triplicate.

The results of the direct RIA binding (Figure 3) showed that ¹²⁵I-labelled mAb Sp3-B4 bound both the synthetic peptide (NANP)₃ and the recombinant Y₁NANP antibody immobilized on microtiter wells. However, the binding to antibody Y₁NANP can be considered more efficient; in molar terms, the estimated ratio of peptide, to antibody was about 50 to 1, assuming that the antibody expresses two copies of the (NANP)₃ epitope per Ig molecule. No binding occurred to either the WT Ig or two irrelevant synthetic peptides, one corresponding to the CDR3 sequence of prototype V_{H}62 and the other to residues YPQVTRGDVFTMPED of vitronectin.

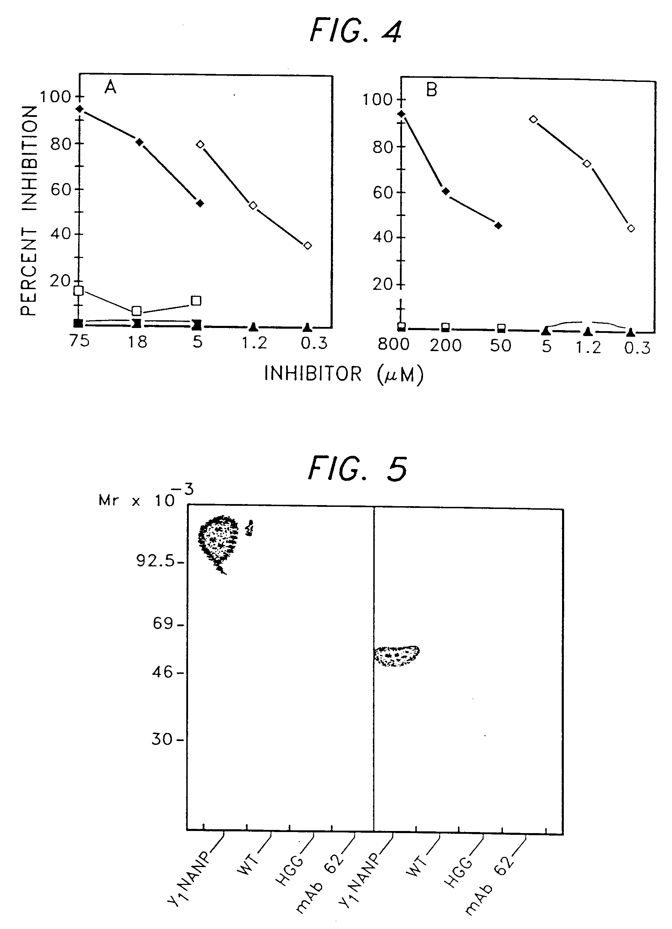

Figure 4 is a Western blot binding of ^{1²5}I-labelled antibody Sp3-B4 to engineered (antigenized) (NANP)₃ epitope in the H chain. Ten »g of purified y1NANP Ig, recombinant WT Ig, native monoclonal antibody 62, and polyclonal human gamma globulins (HGG) (Cohn fraction II, Miles) were loaded onto a 10% SDS-PAGE and electrophoresed at 150 V under nonreducing (left panel) and reducing (right panel) conditions. Resolved proteins or polypeptide chains were transferred from the gel to 0.45-»m nitrocellulose paper. After blotting, the filter was blocked with 10% solution of dry milk in 0.9% NaCI for two hours at room temperature. The sheet was then incubated overnight at +4°C by rocking with ¹²⁵I-labelled antibody Sp3-B4 (40 x 10⁴ cpm/ml) in phosphate-buffered saline, pH 7.3, containing 1% bovine serum albumin and 1% Tween 20. After incubation, the filter was washed extensively, dried and exposed to Kodak XAR-5 film at -70°C for 18 hours. Binding to y1NANP Ig, recombinant WT Ig, antibody 62 and HGG in RIA by the same ¹²⁵I-labelled probe (10⁵ cpm/50»l) was 10,560; 420; 360; and 330 cpm, respectively.

Western blot analysis (Figure 4) showed that ¹²⁵I-labelled mAb Sp3-B4 specifically bound antibody _{Y1}NANP in both the nonreduced (left panel) and reduced (right panel) forms. In the latter, as expected, binding occurred on the H- but not the L-chain, confirming that the engineered (antigenized) _{Y1}NANP antibody bears the (NANP)³ epitope on the H chain. No binding occurred to controls for the H and L chain and the human C region.

A cross-inhibition assay was employed to assess the engineered (antigenized) _{Y1}NANP antibody's relative efficiency in expressing the (NANP)₃ epitope. The synthetic peptide (NANP)₃ and antibody _{Y1}NANP were used to inhibit the binding of ¹²⁵I-labelled mAb Sp3-B4 to either the (NANP)₃ peptide or the _{Y1}NANP antibody immobilized on microtiter plates.

Figure 5 shows results of cross-inhibition of ¹²⁵I-labelled led antibody Sp3-B4 binding to synthetic peptide (NANP) ₃ (panel A) or engineered (antigenized) antibody _{Y1}NANP (panel B) by y1NANP Ig or peptide (NANP)₃. A fixed amount of ¹²⁵I-labelled antibody Sp3-B4 (probe) was mixed vol/vol with decreasing amounts of the various inhibitors diluted in phosphate-buffered saline, pH 7.3, containing 1% bovine serum albumin and 1% Tween 20. The mixture was incubated at +4°C overnight by rocking. Fifty »l of each mixture were incubated on individual polyvinyl microtiter wells coated with either synthetic peptide (NANP)₃ (panel A) or purified engineered (antigenized) _{y1}NANP Ig (panel B). The conditions of coating are as detailed in the legend to Figure 4. The following inhibitors were used: purified y1NANP Ig, WT Ig, and synthetic peptides (NANP)₃, CDR3 and vitronectin. The percentage of inhibition was calculated as follows: $\text{[(average binding of the probe alone) - (average binding of the probe incubated in the presence of inhibitor)]/(average binding of the probe alone) x 100}$ 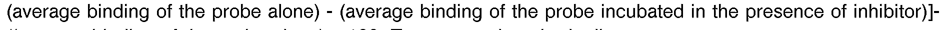 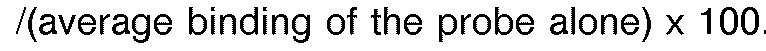 . Tests were done in duplicate.

Figure 5 shows that both the peptide and the engineered (antigenized) antibody efficiently inhibited the binding to both physical forms of the (NANP)₃ epitope, i.e., synthetic peptide and antibody borne. However, whereas the _{y1}NANp antibody was about four times more effective than the peptide itself (panel A) in inhibiting binding to the synthetic peptide, it was approximately 150 times more effective than the peptide in inhibiting binding to the engineered (antigenized) Ig (panel B). The WT Ig and control peptides (CDR3 and vitronectin) caused no inhibition. Thus, when compared with the synthetic peptide it appears that the (NANP)₃ epitope borne on the _{y1}NANP antibody assumes a three-dimensional configuration that in immunological terms more closely mimics that of the active CS protein.

To determine whether the recombinant engineered (antigenized) _{y1}NANP antibody could be Used to induce anti-NANP antibodies, in vivo experiments were performed in rabbits. Two rabbits were immunized with the engineered (antigenized) _{y1}NANP antibody, and two controls receive the WT Ig. As indicated in Table I (on pages 25 and 26) as early as 30 days after the first immunization, both rabbits immunized with the _{y1}NANP antibody produced anti-NANP antibodies detectable by ELISA and RIA. After booster immunizations, the titer rose in both rabbits; the maximal titer was 1/3200 on day 70. Importantly, this antiserum was positive when tested by indirect immunofluorescence on P. falciparum sporozoite showing that the epitope expressed by the _{y1}NANP Ig is indeed mimicking the native antigen. Sera from control rabbits immunized with the WT Ig did not react with the (NANP)₃ peptide immobilized on microtiter wells nor with the parasite. Rabbits of both groups produced an anti-human response as determined by agglutination of red cells coated with human gamma globulin. Rabbit antisera were tested by direct immunofluorescence on P. falciparum (strain Indochina III) dried onto glass slides in the presence of 10% fetal bovine serum.

Studies in vitro using the binding site of a NANP-specific monoclonal antibody as a probe for the protein-surface interaction and in vivo demonstrating that rabbits immunized with the engineered Ig molecule produce anti-NANP antibodies that react with the plasmodium antigen show that the (NANP)₃ epitope expressed by the engineered (antigenized) Ig is both antigenic and immunogenic.

Antibodies were tested for their ability to block sporozoite invasion of liver cells using an in vitro inhibition of sporozoite invasion (ISI) test. (Hollingdale, et al., Journal of Immunology 132:909 (1984). Briefly, serum of DIG fractions were diluted serially and added to Hep62-A16 cell cultures. Sporozoites were incubated with the cell cultures. Attachment and entry of P. falciparum and P. vivax was determined in fixed cultures following staining with species specific monoclonal antibodies. control cultures received either serum or IG fraction from rabbits immunized with the wild type [WT] chimeric protein. the ISI activity of the Ig fraction of rabbits immunized with y1NANP was comparable to the ISI activity of purified Ig elicited by (NANP)₃ peptide coupled to tetanus toxoid in rabbits, further support of the immunogenicity of epitopes presented.

Five groups of mice of different MHC (H2) haplotype (C57BL/6-H2^{b}, BALB/c-H2^{b}, C3H/He-H2^{k}, and SJL-H2^{s}) were immunized intraperitoneally (i.p.) with 50 »g of _{y1}NANP in alum. Booster injections were administrated 30 days later. Serum samples were collected 10 days after the booster injection. Control animals of the same haplotype were immunized with the wild type (WT) chimeric protein. The immunization scheme is as outlined below:
Day -2: Preimmunization Bleed
Day 0: Immunization
Day 29: Post Immunization Bleed
Day 30: 1st Boost
Day 40: Post 1st Boost Bleed
Day 80: Prebleed 2nd Boost and 2nd boost
Day 90: Post 2nd Boost Bleed
Sera were collected 10 days after the second booster immunization. Serum samples from experimental and control groups were tested in ELISA on microtiter plates coated with K K(NANP)₃* (5»g/ml) and as a control other peptides based on amino acid sequences of vitronectin and of the CDR₂ and CDR₃ domains of the parent antibody unrelated to NANP. Shown in Table II as the antibody titers for the individual mice within each strain. The data indicate that the engineered (antigenized) _{y1}NANP can elicit an anti-NANP humoral response in animals of different MCH-haplotype.
* The term "K K(NANP)₃" refers to the coating material placed on the microtiter plates used in the described ELISA assays. The microtiter plates are coated with "K K(NANP)₃" which is three copies of the NANP antigen and lysine (K)

**TABLE II**

| Relative Antibody Titer | | | | | |
|---|---|---|---|---|---|
| Immunization | Mouse | | | | |
| 1 NANP | 25600 | 12800 | 12800 | 12800 | 12800 |
| 2 WT | 3200 | 1600 | 3200 | 6400 | 3200 |
| 3 NANP | 12800 | 12800 | 25600 | 12800 | 12800 |
| 4 WT | 3200 | 1600 | 1600 | 800 | 800 |
| 5 NANP | 6400 | 3200 | 25600 | 12800 | 12800 |
| 6 WT | 800 | 1600 | 1600 | 800 | 1600 |
| 7 NANP | 12800 | 6400 | 6400 | 6400 | 12800 |
| 8 WT | 200 | 1600 | 800 | 800 | 800 |
| 9 NANP | 51200 | 25600 | 51200 | 51200 | 51200 |
| 10 WT | 12800 | 6400 | 6400 | 6400 | 6400 |

Five mice from each strain were immunized with either _{y1}NANP or the Wild Type (WT) Protein. The relative antibody titers for each mouse are given as the reciprocal of serum dilutions. (NANP, Immunized with _{y1}NANP; WT, immunized with wild type chimeric protein).

### EXAMPLE II

For productions of CD4-like antibodies, termed y1CD4, methods analogous to those described in detail in Example I were employed. Briefly, the vector encoding y1CD4 antibodies, comprising amino acid residues 42 through 49 [SFLTKGPS] of human CD4 grafted into the region of the V_{H62} gene, was transfected into J558L cells. Selected cells were screened for antibody production and y1CD4 antibodies were purified from isolates that secrete 30 to 40 »g of antibody per ml. of culture supernatant. SDS-PAGE of y1CD4 under non-reducing conditions showed an apparent molecular weight of about 160 kD. Under reducing conditions, this protein properly resolved into H- and L- chains, demonstrating that insertion of the heterologous CD4 sequence did not inhibit assembly of H- and L- chains.

To confirm the presence of the CD4 sequence in y1CD4, a solid-phase radio-immunoassay (RIA) was employed. Microtiter wells were coated with 5 »g/ml of y1CD4. _{y1}NANP coated wells were used as controls. A series of monoclonal antibodies to CD4 (OKT4, Ortho Pharmaceutical, Rahway, New Jersey) was used as the primary antibody followed by ¹²⁵I-labeled rat anti-mouse secondary antibody. As shown in Table III, OKT4O bound strongly and specifically to y1CD4. Western blot analysis confirmed the results obtained by RIA and further demonstrated that the epitope recognized by OKT4O resides in the H-chain of y1CD4. Lack of binding by other antibodies, particularly OKT4A, ruled out the possibility that residues 42 through 49 of CD4 encompass the Ig-binding site of CD4.

**TABLE III**

| antibody | cpm (X1O⁻³) | |
|---|---|---|
| | v1CD4 | v1NANP |
| 4 | .2 | 3.0 |
| 4A | .2 | 2.8 |
| 4B | 3.1 | 1.0 |
| 4C | 4.2 | .3 |
| 4D | 11.9 | .4 |
| 4F | 4.9 | .3 |

The observation that the V_{H} region of an antibody molecule can be engineered (antigenized) to express 15 amino acid residues containing an epitope of an unrelated molecule shows that the V_{H}/C_{H} polypeptide chain containing the foreign epitope is properly assembled with the endogenous L chain to form a (H₂L₂) tetramer, so it appears that the insertion of this epitope in the CDR3 was tolerated and did not affect the overall Ig framework folding. Based upon the present research, as long as the recombinant epitope is stereochemically compatible with contiguous CDR residues, it can be inserted or substituted for a CDR and can be expected to be exposed at the surface of the molecule, although it cannot be ruled out that the results reported here may be due to the nature of the epitope itself. In the construct described here, the (NANP)₃ sequence is flanked on both sides by the amino acids Val and Pro. Possibly, this helps stabilize the inserted epitope by anchoring it at each end. The large ramification at the Cβ atom and the Cγ-methyl group of the Val residue may hinder the main chain by decreasing its flexibility; the side chain of Pro by curling back to the main chain seizes it, leading to the formation of an almost rigid side chain.

In other terms, neither the molecular environment nor the globular folding of Ig modified the immunologic structure of the (NANP)₃ epitope. From a biological standpoint, the (NANP)₃ epitope engineered into an Ig molecule can be viewed as an idiotope a la carte built into the CDR3 of a host V_{H} domain. Based on what is known of the immunogenicity of idiotypes and the predictable events that follow induction of immunity via the idiotype network [Jerne Ann. Immunol. (Paris) 125, 373 (1974); Cozenave et al., PNAS 74 5122 (1977); Urbain et al., PNAS 74, 5126 (1977); Bona et al., J. Exp. Med 153, 951 (1981)], these results imply that an immune response of predetermined epitope specificity can be dictated in molecular terms and Predicted in vitro. This strategy can be exploited to render a B-cell epitope T-independent, proving its utility not only for analyses of the structure and function of epitopes and Igs but also for the development of new antibody vaccines, for example, as an alternative to peptide based vaccines. Preparation of vaccines may be accomplished using extant methology, already developed for immunoglobulins as such.

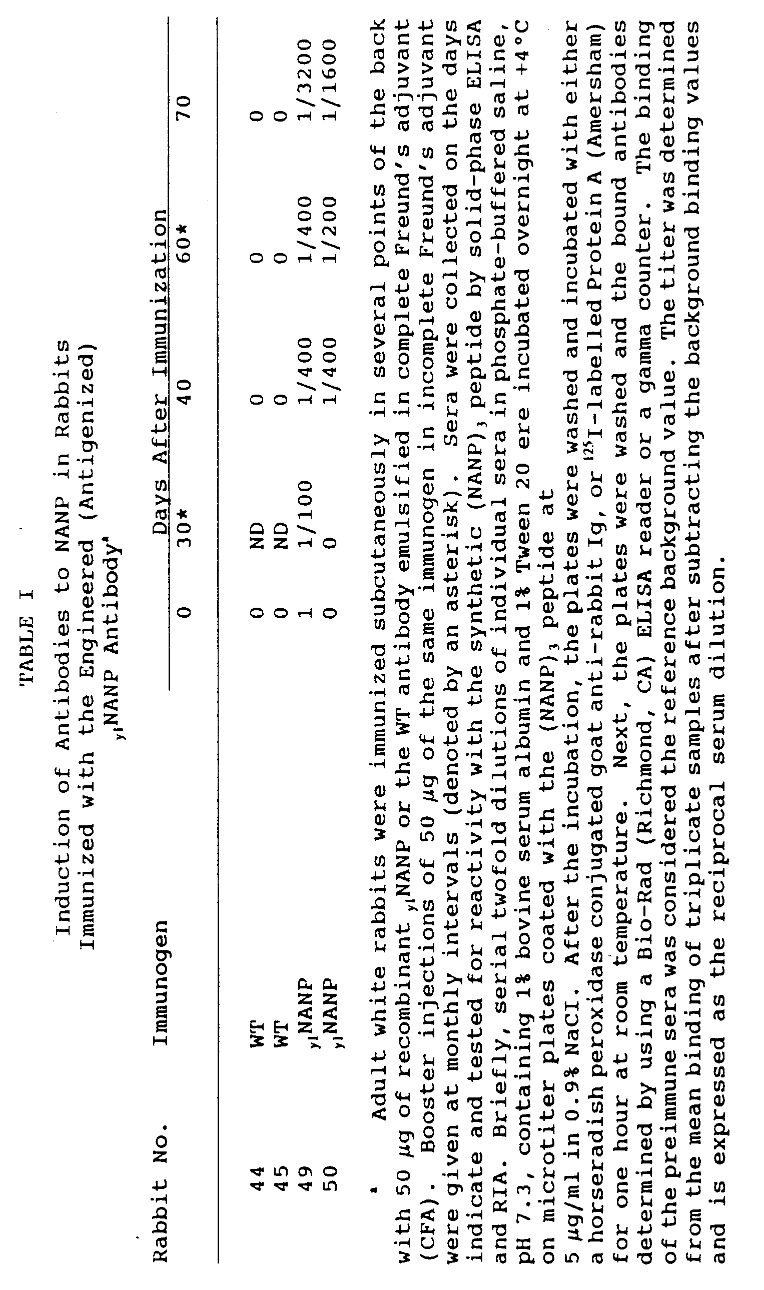

The foregoing description details specific methods that can be employed to practice the present invention. Having detailed specific methods initially used to identify, isolate, characterize, prepare and use the immunoglobulins hereof, and a further disclosure as to specific model entities, the art skilled will well enough know how to devise alternative reliable methods for arriving at the same information and for extending this information to other intraspecies and interspecies related immunoglobulins. Thus, however detailed the foregoing may appear in text, it should not be construed as limiting the overall scope hereof; rather, the ambit of the present invention is to be governed only by the lawful construction of the appended claims.

## Claims (Claims for the following Contracting State(s): AT, BE, CH, DE, DK, FR, GB, IT, LI, LU, NL, SE)

1. An immunoglobulin molecule containing at least one antigenic epitope, not derived from an immunoglobulin molecule, within the N-terminus variable domain thereof, said immunoglobulin molecule having retained functionality in respect of its C-terminus constant region of the heavy chain specific for a particular cell/receptor type, wherein said antigenic epitope is capable of eliciting an antibody response.

2. As a product of recombinant DNA technology, an immunoglobulin according to Claim 1.

3. A heavy chain of an immunoglobulin containing within the N-terminus variable domain thereof at least one antigenic epitope, not derived from an immunoglobulin molecule, wherein said antigenic epitope is capable of eliciting an antibody response.

4. As a product of recombinant DNA technology, the heavy chain according to Claim 3.

5. The heavy chain according to Claim 3 in a form unassembled with its counterpart heavy chain.

6. The heavy chain according to claim 5 in a form unassembled with its associated light chain.

7. A chimeric immunoglobulin molecule according to claim 1.

8. The chimeric immunoglobulin molecule according to Claim 7 made up of hybrid heavy chain composed of sequences selected from both human and non-human species.

9. An immunoglobulin molecule according to Claim 1 wherein the antigenic epitope is the tetrapeptide Asn-Ala-Asn-Pro and is contained within the third complementarity-determining region of its heavy chain.

10. The immunoglobulin molecule according to Claim 9 wherein said tetrapeptide is present in treble form.

11. A pharmaceutical composition containing as an essential principle an immunoglobulin molecule according to Claim 1.

12. The composition according to Claim 11 suitable for administration to a human subject.

13. The composition according to Claim 11 in the form of an administrable vaccine.

14. A DNA molecule that is a recombinant DNA molecule or a cDNA molecule encoding an immunoglobulin molecule according to Claim 1.

15. The DNA molecule according to Claim 14 encoding the heavy-chain of said immunoglobulin.

16. The DNA molecule according to Claim 14 as a synthetic product.

17. An expression vector operatively harboring DNA encoding an immunoglobulin, defined according to Claim 14 or 15.

18. A recombinant host cell transfected with an expression vector according to Claim 17.

19. A process of preparing an immunoglobulin molecule according to Claim 1 which comprises expressing in a recombinant host cell transfecting DNA encoding said immunoglobulin molecule.

20. The process according to Claim 19 wherein said DNA encodes the heavy chain of said immunoglobulin molecule.

21. Use of an immunoglobulin molecule according to claim 1 for the manufacture of a composition useful for building tolerance to or for providing active or passive immunity against an antigen, or for down-regulating hypersensitivity to allergens.

22. The use according to Claim 21 wherein said immunoglobulin molecule is a principle in an administrable pharmaceutical vaccine.

## Claims (Claims for the following Contracting State(s): ES)

1. A process of preparing an immunoglobulin molecule containing at least one antigenic epitope, not derived from an immunoglobulin molecule, within the N-terminus variable domain thereof, said immunoglobulin molecule having retained functionality in respect chain of its C-terminus constant region of the heavy specific for a particular cell/receptor type, wherein said antigenic epitope is capable of eliciting an antibody response which comprises expressing in a recombinant host cell transfecting DNA encoding said immunoglobulin molecule.

2. The process of claim 1, wherein said DNA encodes the heavy chain of an immunogloubulin containing within the N-terminus variable domain thereof at least one antigenic epitope, not derived from an immunoglobulin molecule, wherein said antigenic epitope is capable of eliciting an antibody response.

3. The process of claim 2, wherein the heavy chain is in a form unassembled with its counterpart heavy chain.

4. The process of claim 3, wherein the heavy chain is in a form unassembled with its associated light chain.

5. The process of claim 1, wherein the immunoglobulin molecule is a chimeric immunoglobulin molecule.

6. The process of claim 5, wherein the chimeric immunoglobulin molecule is made up of hybrid heavy chain composed of sequences selected from both human and non-human species.

7. The process of claim 1, wherein the antigenic epitope is the tetrapeptide Asn-Ala-Asn-Pro and is contained within the third complementarity-determining region of its heavy chain.

8. The process of claim 7, wherein said tetrapeptide is present in treble form.

9. A proces for preparing a pharmaceutical composition which comprises introducing as an essential principle an immunoglobulin molecule prepared according to claim 1 into such composition.

10. The process of claim 9, wherein the composition is suitable for administration to a human subject.

11. The process of claim 9, wherein the composition is in the form of an administrable vaccine.

12. Use of a DNA molecule that is a recombinant DNA molecule or a cDNA molecule encoding an immunoglobulin molecule defined according to claim 1, in the process of claim 1.

13. The use of claim 12, wherein said DNA molecule encodes the heavy chain of said immunoglobulin.

14. The use of claim 12, wherein said DNA molecule is a synthetic product.

15. An expression vector operatively harboring a recombinant DNA molecule or a cDNA molecule encoding an immunoglobulin molecule defined according to claim 1.

16. A recombinant host cell transfected with an expression vector according to claim 5.

17. Use of an immunoglobulin molecule defined according to claim 1 for the manufacture of a composition useful for building tolerance to or for providing active or passive immunity against an antigen, or for down-regulation hypersensitivity to allergens.

18. The use according to claim 17, wherein said immunoglobulin molecule is a principle in an administrable pharmaceutical vaccine.

## Patentansprüche (Patentansprüche für folgende(n) Vertragsstaat(en): AT, BE, CH, DE, DK, FR, GB, IT, LI, LU, NL, SE)

1. Immunoglobulinmolekül mit wenigstens einem antigenen Epitop, das nicht vom Immunoglobulinmolekül hergeleitet ist, innerhalb seiner N-terminalen variablen Domäne, welches Immunoglobulinmolekül für einen bestimmten Zell/Rezeptor-Typ spezifische Funktionalität hinsichtlich seiner C-terminalen konstanten Region der schweren Ketten bewahrt hat, wobei das antigene Epitop eine Antikörperantwort auslösen kann.

2. Immunoglobulin nach Anspruch 1 als Produkt rekombinanter DNA-Technologie.

3. Schwere Kette eines Immunoglobulins, welche in ihrer N-terminalen variablen Domäne wenigstens ein antigenes Epitop enthält, das nicht von einem Immunoglobulinmolekül stammt, wobei das antigene Epitop eine Antikörperantwort auslösen kann.

4. Schwere Kette nach Anspruch 3 als Produkt rekombinanter DNA-Technologie.

5. Schwere Kette nach Anspruch 3 in einer nicht mit ihrer komplementären schweren Kette verbundenen Form.

6. Schwere Kette nach Anspruch 5 in einer nicht mit ihrer assoziierten leichten Kette verbundenen Form.

7. Schimäres Immunoglobulinmolekül nach Anspruch 1.

8. Schimäres Immunoglobulinmolekül nach Anspruch 7, aufgebaut aus einer hybriden schweren Kette, die aus sowohl von menschlichen als auch nicht-menschlichen Spezies ausgewählten Sequenzen zusammengesetzt ist.

9. Immunoglobulinmolekül nach Anspruch 1, worin das antigene Eptitop das Tetrapeptid Asn-Ala-Asn-Pro ist und in der dritten komplementaritätsbestimmenden Region seiner schweren Kette enthalten ist.

10. Immunoglobulinmolekül nach Anspruch 9, worin das Tetrapeptid in Tripelform vorliegt.

11. Pharmazeutische Zusammensetzung, die als wesentlichen Wirkstoff ein Immunoglobulinmolekül nach Anspruch 1 enthält.

12. Zusammensetzung nach Anspruch 11, geeignet für die Verabreichung an ein menschliches Wesen.

13. Zusammensetzung nach Anspruch 11 in Form eines verabreichbaren Impfstoffs.

14. DNA-Molekül, das ein rekombinantes DNA-Molekül oder ein cDNA-Molekül ist, welche für ein Immunoglobulinmolekül nach Anspruch 1 kodieren.

15. DNA-Molekül nach Anspruch 14, welches für die schwere Kette des Immunoglobulins kodiert.

16. DNA-Molekül nach Anspruch 14 als synthetisches Produkt.

17. Expressionsvektor, welcher für ein Immunoglobulin gemäß Anspruch 14 oder 15 kodierende DNA aktiv beherbergt.

18. Rekombinante Wirtszelle, transfiziert mit einem Expressionsvektor nach Anspruch 17.

19. Verfahren zur Herstellung eines Immunoglobulinmoleküls nach Anspruch 1 durch Expression transfizierender DNA, die für das Immunoglobulinmolekül kodiert, in einer rekombinanten Wirtszelle.

20. Verfahren nach Anspruch 19, worin die DNA für die schwere Kette des Immunoglobulinmoleküls kodiert.

21. Verwendung eines Immunoglobulinmoleküls nach Anspruch 1 zur Herstellung einer Zusammensetzung zum Aufbau einer Toleranz für oder zur Verleihung aktiver oder passiver Immunität gegen ein Antigen oder zur Verminderung der Hypersensibilität gegenüber Allergenen.

22. Verwendung nach Anspruch 21, worin das Immunoglobulinmolekül ein Wirkstoff in einem verabreichbaren pharmazeutischen Impfstoff ist.

## Patentansprüche (Patentansprüche für folgende(n) Vertragsstaat(en): ES)

1. Verfahren zur Herstellung eines Immunoglobulinmoleküls mit wenigstens einem antigenen Epitop, das nicht vom Immunoglobulinmolekül hergeleitet ist, innerhalb seiner N-terminalen variablen Domäne, welches Immunoglobulinmolekül für einen bestimmten Zell/Rezeptor-Typ spezifische Funktionalität hinsichtlich seiner C-terminalen konstanten Region der schweren Kette bewahrt hat, wobei das antigene Epitop eine Antikörperantwort auslösen kann, durch Expression transfizierender DNA, die für das Immunoglobulinmolekül kodiert, in einer rekombinanten Wirtszelle.

2. Verfahren nach Anspruch 1, worin die DNA für die schwere Kette eines Immunoglobulins kodiert, welche in ihrer N-terminalen variablen Domäne wenigstens ein antigenes Epitop enthält, das nicht von einem Immunoglobulinmolekül stammt, wobei das antigene Epitop eine Antikörperantwort auslösen kann.

3. Verfahren nach Anspruch 2, worin die schwere Kette in einer nicht mit ihrer komplementären schweren Kette verbundenen Form vorliegt.

4. Verfahren nach Anspruch 3, worin die schwere Kette in einer nicht mit ihrer assoziierten leichten Kette verbundenen Form vorliegt.

5. Verfahren nach Anspruch 1, worin das Immunoglobulinmolekül ein schimäres Immunoglobulinmolekül ist.

6. Verfahren nach Anspruch 5, worin das schimäre Immunoglobulinmolekül aus einer hybriden schweren Kette, die aus sowohl von menschlichen als auch nicht-menschlichen Spezies ausgewählten Sequenzen zusammengesetzt ist, aufgebaut ist.

7. Verfahren nach Anspruch 1, worin das antigene Epitop das Tetrapeptid Asn-Ala-Asn-Pro ist und in der dritten komplementaritätsbestimmenden Region der schweren Kette des Immunoglobulins enthalten ist.

8. Verfahren nach Anspruch 7, worin das Tetrapeptid in Tripelform vorliegt.

9. Verfahren zur Herstellung einer pharmazeutischen Zusammensetzung durch Einbringen, als wesentlichen Wirkstoff, eines nach Anspruch 1 hergestellten Immunoglobulinmoleküls in solch eine Zusammensetzung.

10. Verfahren nach Anspruch 9, worin die Zusammensetzung zur Verabreichung an ein menschliches Wesen geeignet ist.

11. Verfahren nach Anspruch 9, worin die Zusammensetzung in Form eines verabreichbaren Impfstoffes vorliegt.

12. Verwendung eines DNA-Moleküls, das ein rekombinantes DNA-Molekül oder ein cDNA-Molekül ist, welche für ein Immunoglobulinmolekül nach Anspruch 1 kodieren, im Verfahren nach Anspruch 1.

13. Verwendung nach Anspruch 12, worin das DNA-Molekül für die schwere Kette des Immunoglobulins kodiert.

14. Verwendung nach Anspruch 12, worin das DNA-Molekül ein synthetisches Produkt ist.

15. Expressionsvektor, welcher ein für ein Immunoglobulinmolekül nach Anspruch 1 kodierendes rekombinantes DNA-Molekül oder cDNA-Molekül aktiv beherbergt.

16. Rekombinante Wirtzelle, transfiziert mit einem Expressionsvektor nach Anspruch 15.

17. Verwendung eines Immunoglobulinmoleküls nach Anspruch 1 zur Herstellung einer Zusammensetzung zum Aufbau einer Toleranz für oder zur Verleihung aktiver oder passiver Immunität gegen ein Antigen oder zur Verminderung der Hypersensibilität gegenüber Allergenen.

18. Verwendung nach Anspruch 17, worin das Immunoglobulinmolekül ein Wirkstoff in einem verabreichbaren pharmazeutischen Impfstoff ist.

## Revendications (Revendications pour l'(les) Etat(s) contractant(s) suivant(s): AT, BE, CH, DE, DK, FR, GB, IT, LI, LU, NL, SE)

1. Molécule d'immunoglobuline contenant au moins un épitope antigénique, ne provenant pas d'une molécule d'immunoglobuline, dans son domaine variable N-terminal, ladite molécule d'immunoglobuline ayant une fonctionnalité conservée concernant sa région constante C-terminale de la chaîne lourde spécifique d'un type de cellule/récepteur particulier, dans laquelle ledit épitope antigénique est capable de déclencher une réponse anticorps.

2. Immunoglobuline selon la revendication 1 en tant que produit de la technologie de recombinaison d'ADN.

3. Chaîne lourde d'immunoglobuline contenant dans son domaine variable N-terminal au moins un épitope antigénique, ne provenant pas d'une molécule d'immunoglobuline, dans laquelle ledit épitope antigénique est capable de déclencher une réponse anticorps.

4. Chaîne lourde selon la revendication 3 en tant que produit de la technologie de recombinaison d'ADN.

5. Chaîne lourde selon la revendication 3 sous une forme non assemblée avec sa chaîne lourde homologue.

6. Chaîne lourde selon la revendication 5 sous une forme non assemblée avec sa chaîne légère associée.

7. Molécule d'immunoglobuline chimérique selon la revendication 1.

8. Molécule d'immunoglobuline chimérique selon la revendication 7 constituée par une chaîne lourde hybride composée de séquences choisies parmi les espèces humaines et non humaines.

9. Molécule d'immunoglobuline selon la revendication 1 dans laquelle l'épitope antigénique est le tétrapeptide Asn-Ala-Asn-Pro et est contenu dans la troisième région de détermination de complémentarité de sa chaîne lourde.

10. Molécule d'immunoglobuline selon la revendication 9 dans laquelle ledit tétrapeptide est présent sous forme triple.

11. Composition pharmaceutique contenant comme principe essentiel une molécule d'immunoglobuline selon la revendication 1.

12. Composition selon la revendication 11 qui convient pour l'administration à un sujet humain.

13. Composition selon la revendication 11 sous la forme d'un vaccin administrable.

14. Molécule d'ADN qui est une molécule d'ADN recombinée ou une molécule d'ADNc codant une molécule d'immunoglobuline selon la revendication 1.

15. Molécule d'ADN selon la revendication 14 qui code la chaîne lourde de ladite immunoglobuline.

16. Molécule d'ADN selon la revendication 14 en tant que produit synthétique.

17. Vecteur d'expression contenant de manière active un ADN codant une immunoglobuline défini selon la revendication 14 ou 15.

18. Cellule hôte recombinée transfectée avec un vecteur d'expression selon la revendication 17.

19. Procédé de préparation d'une molécule d'immunoglobuline selon la revendication 1 qui comprend l'expression dans une cellule hôte recombinée d'un ADN de transfection codant ladite molécule d'immunoglobuline.

20. Procédé selon la revendication 19 dans lequel ledit ADN code la chaîne lourde de ladite molécule d'immunoglobuline.

21. Utilisation d'une molécule d'immunoglobuline selon la revendication 1 pour la fabrication d'une composition utile pour conférer une tolérance vis-à-vis de ou pour procurer une immunité active ou passive contre un antigène, ou pour la régulation négative de l'hypersensibilité aux allergènes.

22. Utilisation selon la revendication 21 dans laquelle ladite molécule d'immunoglobuline est un principe dans un vaccin pharmaceutique administrable.

## Revendications (Revendications pour l'(les) Etat(s) contractant(s) suivant(s): ES)

1. Procédé de préparation d'une molécule d'immunoglobuline contenant au moins un épitope antigénique, ne provenant pas d'une molécule d'immunoglobuline, dans son domaine variable N-terminal, ladite molécule d'immunoglobuline ayant une fonctionnalité conservée concernant sa région constante C-terminale de la chaîne lourde spécifique d'un type de cellule/ récepteur particulier, dans lequel ledit épitope antigénique est capable de déclencher une réponse anticorps, qui comprend l'expression dans une cellule hôte recombinée d'un ADN de transfection codant ladite molécule d'immunoglobuline.

2. Procédé selon la revendication 1, dans lequel ledit ADN code la chaîne lourde d'une immunoglobuline contenant dans son domaine variable N-terminal au moins un épitope antigénique ne provenant pas d'une molécule d'immunoglobuline, dans lequel ledit épitope antigénique est capable de déclencher une réponse anticorps.

3. Procédé selon la revendication 2, dans lequel la chaîne lourde est sous une forme non assemblée avec sa chaîne lourde homologue.

4. Procédé selon la revendication 3, dans lequel la chaîne lourde est sous une forme non assemblée avec sa chaîne légère associée.

5. Procédé selon la revendication 1, dans lequel la molécule d'immunoglobuline est une molécule d'immunoglobuline chimérique.

6. Procédé selon la revendication 5, dans lequel la molécule d'immunoglobuline chimérique est constituée par une chaîne lourde hybride composée de séquences choisies parmi les espèces humaine et non humaines.

7. Procédé selon la revendication 1, dans lequel épitope antigénique est le tétrapeptide Asn-Ala-Asn-Pro et est contenu dans la troisième région de détermination de complémentarité de sa chaîne lourde.

8. Procédé selon la revendication 7, dans lequel ledit tétrapeptide est présent sous forme triple.

9. Procédé de préparation d'une composition pharmaceutique qui comprend l'introduction comme principe essentiel dans une telle composition d'une molécule d'immunoglobuline préparée selon la revendication 1.

10. Procédé selon la revendication 9, dans lequel la composition convient pour l'administration à un sujet humain.

11. Procédé selon la revendication 9, dans lequel la composition est sous forme d'un vaccin administrable.

12. Utilisation dans le procédé selon la revendication 1 d'une molécule d'ADN qui est une molécule d'ADN recombinée ou une molécule d'ADNc codant une molécule d'immunoglobuline définie selon la revendication 1.

13. Utilisation selon la revendication 12, dans laquelle ladite molécule d'ADN code la chaîne lourde de ladite immunoglobuline.

14. Utilisation selon la revendication 12, dans laquelle ladite molécule d'ADN est un produit synthétique.

15. Vecteur d'expression contenant de manière active une molécule d'ADN recombinée ou une molécule d'ADNc codant une molécule d'immunoglobuline définie selon la revendication 1.

16. Cellule hôte recombinée transfectée avec un vecteur d'expression selon la revendication 15.

17. Utilisation d'une molécule d'immunoglobuline définie selon la revendication 1 pour la fabrication d'une composition utile pour conférer une tolérance vis-à-vis de ou pour procurer une immunité active ou passive contre un antigène, ou pour la régulation négative de l'hypersensibilité aux allergènes.

18. Utilisation selon la revendication 17, dans laquelle ladite molécule d'immunoglobuline est un principe dans un vaccin pharmaceutique administrable.
